# FASCICULE DE BREVET EUROPEEN

(11) **EP 4 120 946 B1**
(45) Date de publication et mention de la délivrance du brevet: **08.05.2024**
(21) Numéro de dépôt: 21711598.9
(22) Date de dépôt: 18.03.2021
(51) Int. Cl.: A61B 34/10, A61B 17/70

(54) **PROCÉDÉ ET DISPOSITIF D'AIDE À UNE INTERVENTION INVASIVE SUR UN ORGANE HUMAIN OU ANIMAL**
VERFAHREN UND VORRICHTUNG ZUR UNTERSTÜTZUNG EINES INVASIVEN EINGRIFFS AN EINEM MENSCHLICHEN ODER TIERISCHEN ORGAN
METHOD AND DEVICE FOR ASSISTING AN INVASIVE PROCEDURE ON A HUMAN OR ANIMAL ORGAN

(30) Priorité: 18.03.2020 FR 2002648
(43) Date de publication de la demande: 25.01.2023
(73) Titulaire: Pytheas Navigation, 13008 Marseille (FR)
(72) Inventeur: GLARD, Yann, 13008 Marseille (FR)
(74) Mandataire: Santarelli
(86) Numéro de dépôt international: PCT/EP2021/056898
(87) Numéro de publication internationale: WO 2021/185940

(56) Documents cités:
- US-A1- 2019 046 269
- Brendan Polley: "3D Slicer - Pedicle Screw Surgical Simulator", Youtube, 11 janvier 2014 (2014-01-11), page 4 pp., XP054981158, Extrait de l'Internet: URL:https://www.youtube.com/watch?v=XN3Tp8 jaYdQ [extrait le 2020-12-01]
- Surgimap: "Surgimap Desktop Software: Pedicle Screw Planning", Youtube, 11 juillet 2017 (2017-07-11), page 3 pp., XP054981159, Extrait de l'Internet: URL:https://www.youtube.com/watch?v=8BITlG _09h8 [extrait le 2020-12-01]
- SOLITRO GIOVANNI F ET AL: "Innovative approach in the development of computer assisted algorithm for spine pedicle screw placement", MEDICAL ENGINEERING & PHYSICS, vol. 38, no. 4, 1 avril 2016 (2016-04-01), pages 354-365, XP029458088, ISSN: 1350-4533, DOI: 10.1016/J.MEDENGPHY.2016.01.005

## Description

La présente invention concerne un procédé et un dispositif d'aide à une intervention invasive sur un organe humain ou animal, par exemple pour planifier la pose d'implants rachidiens.

La présente invention est utilisable, en particulier, pour une chirurgie en rapport avec une partie anatomique humaine ou animale sensiblement rigide (c'est-à-dire une partie anatomique qui ne se déforme pas ou se déforme peu sous une pression modérée). A titre d'illustration, des modes de réalisation de l'invention concernent la préparation de la chirurgie rachidienne, par exemple la préparation de l'insertion de vis pédiculaires dans une ou plusieurs vertèbres.

La structure squelettique interne d'un mammifère, humain ou animal, est composée d'une centaine d'os. La colonne vertébrale est une chaine d'os appelée vertèbres permettant une certaine souplesse et des mouvements, tout en protégeant les structures nerveuses et vasculaires qui se trouvent à l'intérieur et autour de la colonne vertébrale. La colonne vertébrale commence à la base du crâne et s'étend jusqu'au bassin. Elle est composée de quatre régions : cervicale, thoracique, lombaire et sacrée.

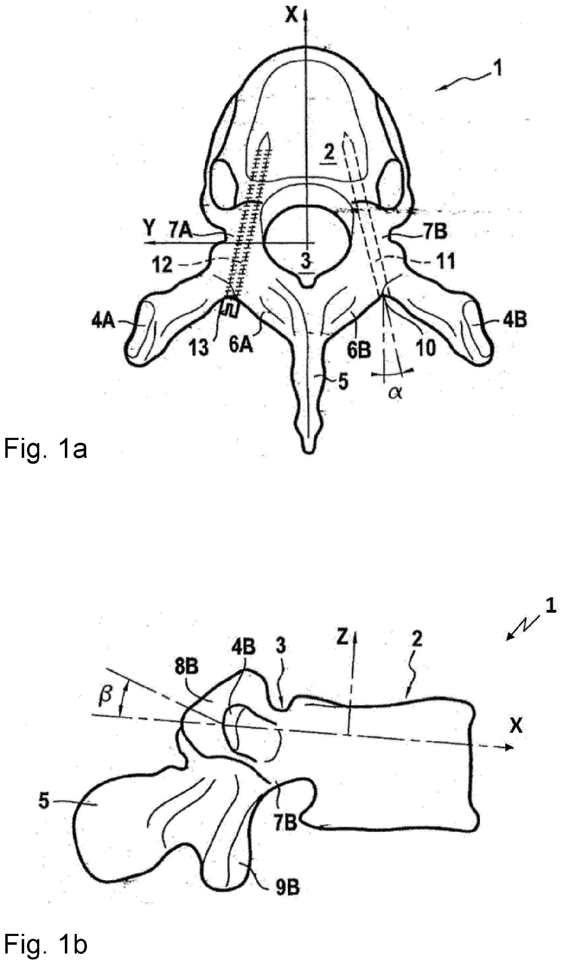

Les figures 1A et 1B représentent respectivement une vue de dessus et une vue de côté d'une vertèbre thoracique 1. Comme illustré, la vertèbre 1 comprend un corps vertébral 2 orienté vers l'avant, un foramen vertébral 3 en forme de trou permettant à la moelle épinière de passer, deux processus transverses 4A et 4B orientés vers l'arrière et vers l'extérieur, un processus épineux 5 entre les processus transverses 4A et 4B et orienté vers le bas, deux lames 6A et 6B qui connectent les processus transverses 4A et 4B au processus épineux 5, deux pédicules 7A et 7B qui connectent le corps vertébral 2 aux processus transverses 4A et 4B, deux facettes articulaires supérieures 8A (non représentée) et 8B et deux facettes articulaires inférieures 9A (non représentée) et 9B qui permettent l'articulation de la vertèbre 1 avec une vertèbre adjacente (elles sont empilées les unes sur les autres).

L'alignement vertébral normal ou idéal peut être perturbé suite à un traumatisme ou une maladie (par exemple une scoliose). Les vertèbres peuvent alors pivoter autour de trois axes (X, Y, Z), nécessitant parfois une intervention chirurgicale afin de corriger les anomalies et retrouver un alignement vertébral idéal ou, à tout le moins, amélioré.

Dans ce cas, au moins deux vertèbres adjacentes sont généralement fusionnées l'une à l'autre par un procédé dans lequel un chirurgien ouvre le patient, généralement par le dos, détermine un point d'entrée 10 et perce des trous 11 dans les pédicules 7A et 7B de la vertèbre 1 et d'une ou plusieurs autres vertèbres voisines. Les trous sont percés avec un angle axial alpha α (l'angle par rapport au plan vertical XZ) et un angle sagittal beta β (l'angle par rapport au plan XY), comme illustré sur les figures 1A et 1B respectivement.

Des vis pédiculaires 12 comprenant des extrémités 13 en forme de U (mais potentiellement avec d'autres formes) peuvent alors être insérées dans les trous 11. Pour des raisons de clarté de la figure 1A, un seul point d'entrée 10, trou 11, vis pédiculaire 12, et extrémité 13 sont montrés.

Chaque extrémité 13 reçoit un élément de liaison (non montré), par exemple une tige, qui permet de relier plusieurs vis (de plusieurs vertèbres) entre elles et ainsi de réduire la déformation et de fusionner des vertèbres entre elles. Les trous 11 percés et les vis pédiculaires 12 placées dans les vertèbres doivent être soigneusement positionnés et alignés afin de ne pas blesser les structures nerveuses et vasculaires adjacentes, voire provoquer la mort du patient. En cas de mauvais positionnement des vis, il faut procéder à une seconde opération, entrainant des coûts et des risques supplémentaires.

Des systèmes de guidage ont été développés pour aider le chirurgien à percer les trous 11 dans la vertèbre 1 et placer précisément les vis pédiculaires 12.

Par ailleurs, il existe des solutions pour déterminer, durant une phase préopératoire, les positions souhaitables des vis pédiculaires. De telles solutions se basent généralement sur des images radiographiques, permettant au praticien de déterminer une position et une orientation théoriques optimales pour chaque vis.

Ainsi, par exemple, l'article intitulé « A novel cost-effective computer-assisted imaging technology for accurate placement of thoracic pedicle screws », Y. Abe et al., J Neurosurg : Spine, volume 15, novembre 2011, décrit une solution pour aider un praticien à poser des vis pédiculaires. Selon cette solution, la position et l'orientation d'une vis pédiculaire sont déterminées dans une étape d'analyse 3D de données obtenues durant une phase préopératoire. Après avoir défini cette position et cette orientation, le point d'entrée de la vis pédiculaire dans la vertèbre en est déduit. En utilisant des points de repère sur la vertèbre (par exemple, les processus transversaux, les processus épineux et/ou les pédicules), le praticien peut, lors de l'opération, déterminer le point d'entrée sur la vertèbre et, en fonction du point d'entrée ainsi déterminé et de l'orientation de la vis pédiculaire déterminée lors de la phase préopératoire, percer la vertèbre pour poser la vis pédiculaire.

Cependant, si de tels procédés permettent la mise en place de vis pédiculaires de façon simplifiée et économique, elles ne permettent pas toujours d'atteindre la précision souhaitée.

Il y a ainsi un besoin pour un procédé et un dispositif d'aide à une intervention invasive sur un organe humain ou animal sensiblement rigide, par exemple pour planifier la pose d'implants rachidiens, permettant d'améliorer la précision spatiale de l'intervention.

La présente invention répond à cette problématique.

Par ailleurs, la vidéo Brendan Polley: "3D Slicer Pedicle Screw Surgical Simulator",Youtube, 11 janvier 2014 (2014.01.11), extraite de l'Internet: https://www.youtube.com/watch?v=XN3Tp8jaYdQ [extraite le 2020.12.01] divulgue un simulateur conçu comme un module Python pour le logiciel libre d'analyse d'images, 3D Slicer. Le simulateur permet aux utilisateurs d'importer des tomodensitogrammes ou des IRM spécifiques à un patient sous forme d'ensembles de données DICOM, et peut être entièrement intégré aux serveurs d'archivage et de communication d'images (PACS). Les utilisateurs peuvent ensuite utiliser des vues orthographiques en 2D ainsi que des rendus en 3D de la colonne vertébrale d'un patient pour prendre des mesures en vue d'une intervention chirurgicale. Les utilisateurs sont ensuite immergés dans un environnement interactif, dans lequel ils peuvent manipuler des modèles 3D de vis pédiculaires et les insérer dans le modèle de la colonne vertébrale du patient. Le placement de la vis peut alors être évalué en calculant le pourcentage d'os cortical et spongieux en contact avec la surface de chaque vis, et affiché graphiquement. En outre, la position de chaque modèle de vis est comparée aux données de tomodensitométrie du patient afin de cartographier les intensités des pixels de l'unité Hounsfield directement sur la surface du modèle de vis.

### Exposé de l'invention

A cet effet, l'invention propose de déterminer un point d'entrée d'un élément, sur un modèle 3D de l'organe dans lequel doit être inséré cet élément, puis déterminer l'orientation selon laquelle doit être inséré cet élément afin d'optimiser la position et l'orientation de celui-ci.

Il est ainsi proposé un procédé d'aide à une intervention invasive sur un organe humain ou animal, ladite intervention comprenant l'insertion d'au moins un élément dans ledit organe, le procédé comprenant (1) l'obtention d'un modèle tridimensionnel d'au moins une partie dudit organe, (2) la détermination sur le modèle tridimensionnel obtenu d'au moins un point d'entrée dudit au moins un élément et (3) la détermination d'un axe d'insertion dudit au moins un élément dans ledit organe, en fonction dudit modèle tridimensionnel, dudit au moins un point d'entrée déterminé et d'une pluralité d'axes distincts passant par ledit au moins un point d'entrée.

Le procédé selon l'invention permet ainsi un positionnement précis d'un élément en choisissant tout d'abord le point d'entrée de cet élément selon des critères assurant, en pratique, une détermination facile de ce point d'entrée, puis en choisissant l'orientation selon laquelle il doit être inséré.

Selon un mode de réalisation particulier, le procédé comprend en outre la construction dudit modèle tridimensionnel. Une telle construction peut être réalisée, par exemple, par tomographie numérique.

Toujours selon un mode de réalisation particulier, le procédé comprend en outre l'affichage d'une représentation d'au moins une partie dudit modèle tridimensionnel, l'affichage d'au moins une partie dudit modèle tridimensionnel permettant l'affichage et/ou la détermination d'au moins un point d'entrée.

Toujours selon un mode de réalisation particulier, au moins deux points d'entrées sont déterminés, ladite pluralité d'axes étant définie par l'intersection d'un premier plan comprenant une droite passant par lesdits au moins deux points d'entrée et d'un second plan comprenant un point d'entrée. Ledit second plan peut comprendre une droite perpendiculaire audit premier plan.

Toujours selon un mode de réalisation particulier, le procédé comprend en outre l'affichage d'une vue en coupe dudit modèle tridimensionnel selon ledit premier plan, ledit premier plan formant un premier angle par rapport à une première référence liée audit modèle tridimensionnel.

Toujours selon un mode de réalisation particulier, le procédé comprend une modification de la valeur dudit premier angle, l'affichage d'une vue en coupe selon ledit premier plan étant adapté à ladite modification dudit premier angle.

Toujours selon un mode de réalisation particulier, le procédé comprend en outre l'affichage d'une vue en coupe dudit modèle tridimensionnel selon ledit second plan, ledit second plan formant un second angle par rapport à une seconde référence liée audit modèle tridimensionnel.

Toujours selon un mode de réalisation particulier, le procédé comprend une modification de la valeur dudit second angle, l'affichage d'une vue en coupe selon ledit second plan étant adapté à ladite modification dudit second angle.

Toujours selon un mode de réalisation particulier, le procédé comprend le déplacement d'au moins un point d'entrée défini sur ledit modèle tridimensionnel.

Toujours selon un mode de réalisation particulier, le procédé comprend une mesure d'un diamètre et/ou d'une longueur d'un élément à insérer selon un point d'entrée déterminé, un axe d'insertion déterminé et ledit modèle tridimensionnel.

Ledit organe est, par exemple, une vertèbre et ledit élément à insérer est, par exemple, une vis pédiculaire.

L'invention vise également un dispositif d'aide à une intervention invasive sur un organe humain ou animal, le dispositif comprenant une unité de traitement configurée pour effectuer chacune des étapes du procédé décrit précédemment.

Un programme informatique, mettant en oeuvre tout ou partie du procédé décrit ci-avant, installé sur un équipement préexistant, est en lui-même avantageux, dès lors qu'il fournit une assistance pour aider un utilisateur à identifier une position souhaitée d'un élément dans un organe.

Ainsi, la présente invention vise également un programme informatique comportant des instructions pour la mise en oeuvre du procédé précédemment décrit, lorsque ce programme est exécuté par un processeur.

Ce programme peut utiliser n'importe quel langage de programmation (par exemple, un langage objet ou autre) et être sous la forme d'un code source interprétable, d'un code partiellement compilé ou d'un code totalement compilé.

Un autre aspect concerne un support de stockage non-transitoire d'un programme exécutable par ordinateur, comprenant un ensemble de données représentant un ou plusieurs programmes, lesdits un ou plusieurs programmes comprenant des instructions pour, lors de l'exécution desdits un ou plusieurs programmes par un ordinateur comprenant une unité de traitement couplée de manière opérationnelle à des moyens mémoire et à un module d'interface entrées/sorties, pour exécuter tout ou partie du procédé décrit ci-avant.

### Brève description des dessins

D'autres caractéristiques, détails et avantages de l'invention apparaîtront à la lecture de la description détaillée ci-après. Celle-ci est purement illustrative et doit être lue en regard des dessins annexés, sur lesquels :
**Fig. 1**
   La figure 1A [Fig. 1A] et la figure 1B [Fig. 1B] représentent respectivement une vue de dessus et une vue de côté d'une vertèbre thoracique ;
**Fig. 2**

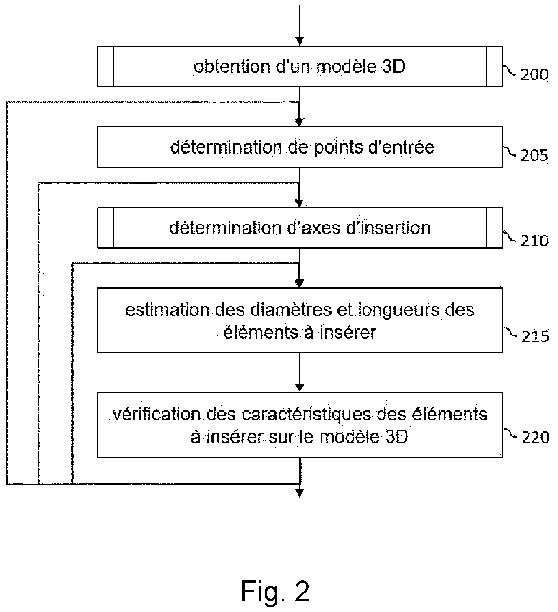

   La figure 2 [Fig. 2] illustre un exemple d'étapes pouvant être mises en oeuvre dans un outil d'aide à une intervention invasive sur un organe humain ou animal selon l'invention ;
**Fig. 3**

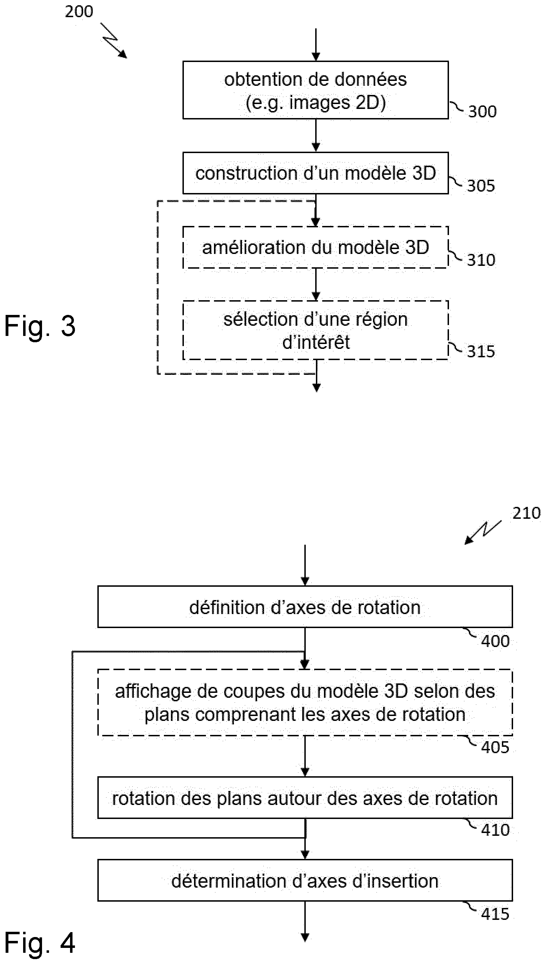

   La figure 3 [Fig. 3] illustre un exemple d'étapes pour obtenir un modèle 3D d'un organe humain ou animal ou d'une portion de celui-ci ;
**Fig. 4**
   La figure 4 [Fig. 4] illustre un exemple d'étapes pour déterminer un axe d'insertion d'un élément dans un organe humain ou animal, par exemple un axe d'un support tel qu'une vis pédiculaire ;
**Fig. 5**

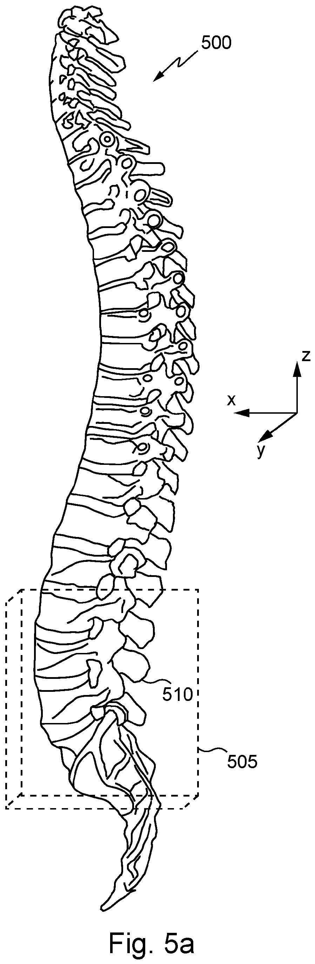

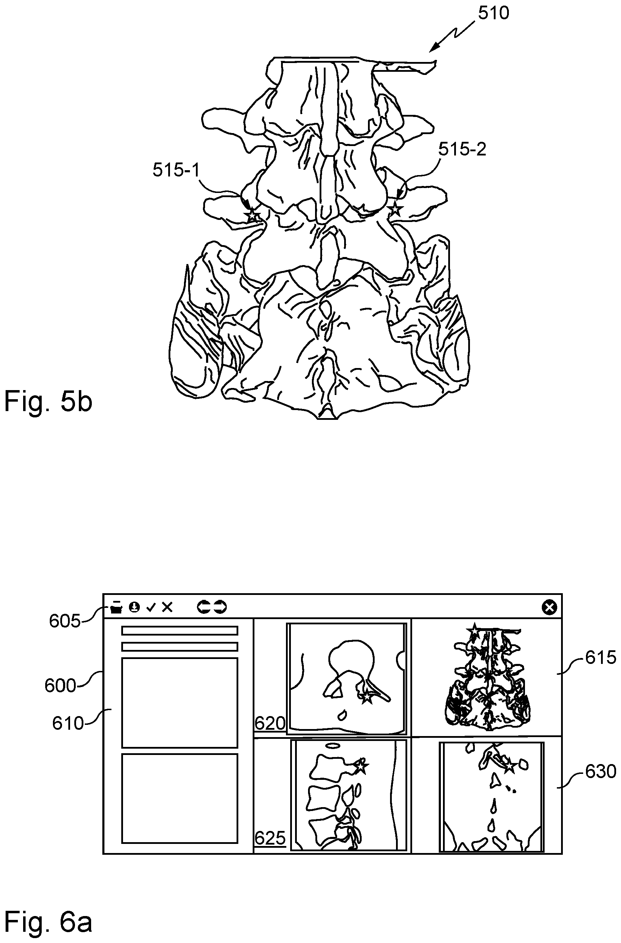

   La figure 5A [Fig. 5A] et la figure 5B [Fig. 5B] illustrent un exemple de modèle 3D d'une colonne vertébrale, permettant à un utilisateur de déterminer un ou plusieurs points d'entrée puis de déterminer un ou plusieurs axes d'insertion de vis pédiculaires ;
**Fig. 6**

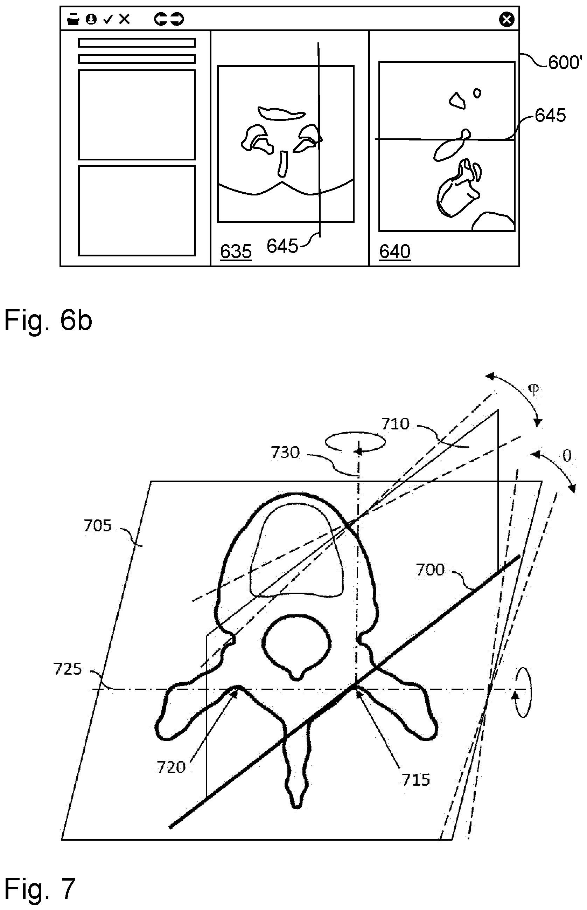

   La figure 6A [Fig. 6A] et la figure 6B [Fig. 6B] représentent un exemple d'une interface graphique pouvant être utilisée par un utilisateur pour déterminer des points d'entrée et des axes d'insertion d'éléments tels que des vis pédiculaires ;
**Fig. 7**
   La figure 7 [Fig. 7] illustre schématiquement un exemple d'une solution pour déterminer un axe d'insertion d'un élément dans un organe, par exemple d'une vis pédiculaire dans une vertèbre ;
**Fig. 8**

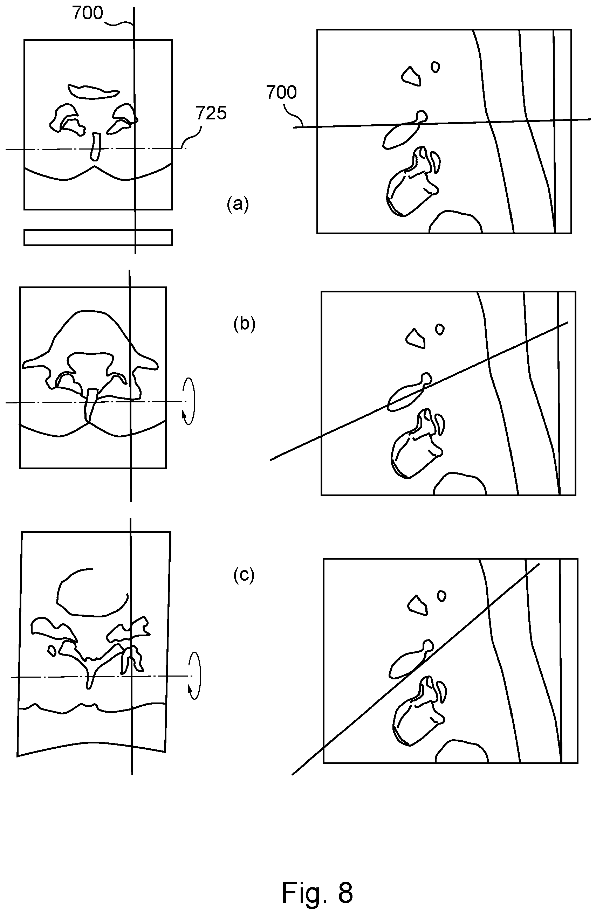

   La figure 8 [Fig. 8] illustre un exemple de vues en coupe obtenues lors de la rotation d'un plan passant par deux points d'entrée, selon le mécanisme illustré sur la figure 7 ;
**Fig. 9**

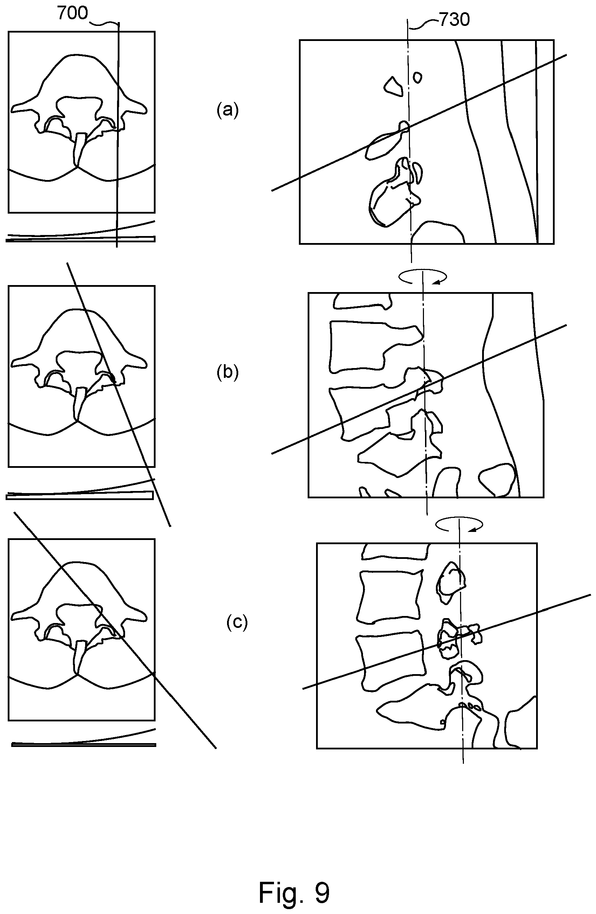

   La figure 9 [Fig. 9] illustre un exemple de vues en coupe obtenues lors de la rotation d'un plan perpendiculaire à un plan passant par deux points d'entrée, selon le mécanisme illustré sur la figure 7 ; et
**Fig. 10**

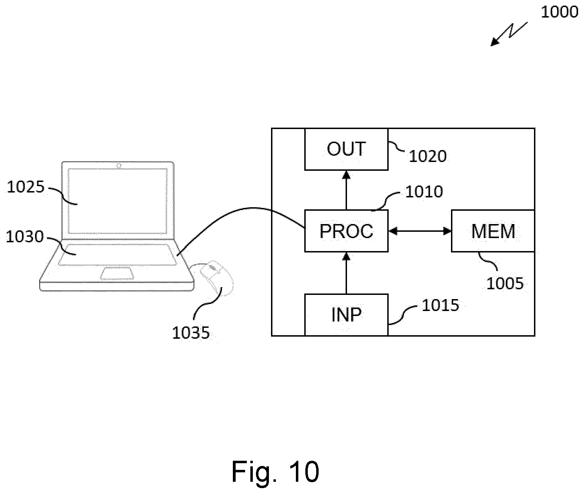

   La figure 10 [Fig. 10] représente un exemple de dispositif de traitement de l'information permettant de mettre en oeuvre le procédé selon des modes de réalisation de l'invention.

### Description détaillée

Selon des modes de réalisation, un outil d'aide à une intervention invasive sur un organe humain ou animal selon l'invention vise tout d'abord à déterminer un point d'entrée dans l'organe puis, à partir de ce point d'entrée, à déterminer une position et une orientation avantageuse d'un élément à insérer. En choisissant un point d'entrée pouvant être facilement identifié lors de l'intervention, il est possible de positionner précisément un élément dans l'organe. En effet, les inventeurs ont observé qu'il était souvent préférable de choisir un point d'entrée non optimal et de positionner précisément un élément plutôt que déterminer une position optimale d'un élément et en déduire un point d'entrée pouvant se révéler, en pratique, difficile à déterminer.

La figure 2 illustre un exemple d'étapes pouvant être mise en oeuvre dans un outil d'aide à une intervention invasive sur un organe humain ou animal selon l'invention.

Comme illustré, une première étape a pour objet l'obtention d'un modèle numérique tridimensionnel (appelé ci-après modèle 3D) d'un organe humain ou animal, ou d'une portion de celui-ci, dans lequel l'intervention doit avoir lieu (étape 200). Un exemple d'obtention d'un tel modèle 3D est décrit en référence à la figure 3. Selon des modes de réalisation, il s'agit d'un modèle permettant une visualisation surfacique, c'est-à-dire d'une enveloppe de l'organe considéré, comprenant sa surface externe et, le cas échéant, ses orifices ou évidements. Naturellement, le modèle 3D peut être plus complexe et comprendre d'autres informations telles que des représentations de terminaisons nerveuses, vaisseaux sanguins, etc.

Dans une étape suivante, un ou plusieurs points d'entrée sont déterminés sur le modèle 3D (étape 205) ou à partir de celui-ci, par exemple sur des vues en coupe du modèles 3D. Ils peuvent être déterminés automatiquement ou par un utilisateur, par exemple sur une représentation graphique du modèle 3D ou d'une portion de celui-ci. Lorsque le ou les points d'entrée sont déterminés par un utilisateur sur une représentation graphique du modèle 3D, une application de visualisation permettant de modifier l'angle de vue de l'organe est de préférence utilisée. Une telle application peut effectuer des opérations de changement d'échelle, rotation selon plusieurs axes et déplacement selon plusieurs directions. Un exemple de représentations d'un modèle 3D et d'une portion de celui-ci est illustré sur les figures 5A et 5B. Ces représentations permettent de déterminer un point d'entrée. Comme illustré, il est, de préférence, possible de sélectionner une portion du modèle (aussi appelée région d'intérêt), par exemple à l'aide d'un cube, d'un cylindre ou de tout autre forme tridimensionnelle dont, en particulier, la forme, la taille, la position et/ou l'orientation peuvent être ajustées par un utilisateur ou de façon automatique.

Selon d'autres modes de réalisation, un point d'entrée est déterminé à partir de vues en coupe du modèle 3D, par exemple de deux vues en coupe selon des plans perpendiculaires comprenant un même point pouvant être sélectionné comme un point d'entrée. Selon ces modes de réalisation, ce point pouvant être sélectionné comme un point d'entrée peut être déplacé sur la surface du modèle 3D, c'est-à-dire sur des lignes de coupe des vues en coupe, par exemple à l'aide d'une molette de souris ou de touches d'un clavier. Selon d'autres modes de réalisation, le ou les points d'entrée peuvent être sélectionnés sur la surface du modèle 3D et/ou dans l'organe, sous la surface du modèle 3D et à proximité de cette dernière. En effet, il arrive, en pratique, qu'un praticien supprime une partie de l'organe considéré, par exemple un morceau d'os, pour sélectionner un ou plusieurs points d'entrée.

Pour déterminer automatiquement un point d'entrée, une analyse du modèle 3D ou d'une portion du modèle 3D peut être effectuée pour identifier des points remarquables, faciles à identifier durant une intervention, et à partir desquels le point d'entrée peut être défini. De tels points remarquables sont, par exemple, les processus transverses et le processus épineux. A titre d'illustration, une telle analyse peut être effectuée par un algorithme d'intelligence artificielle entrainé avec des modèles 3D sur lesquels des points remarquables ont été placés par un utilisateur ou ont été validés par un utilisateur.

Dans une étape suivante, la position et l'orientation d'un ou plusieurs éléments à insérer dans l'organe sont déterminées, à partir du ou des points d'entrée précédemment choisis (étape 210). Selon des modes de réalisation, de telles position et orientation correspondent à un axe d'un support, s'étendant à partir du point d'entrée. Cette position et cette orientation peuvent être déterminées automatiquement à partir de contraintes prédéterminées ou être déterminées par un utilisateur, par exemple sur une représentation graphique du modèle 3D ou d'une portion de celui-ci. Un exemple de détermination d'une position par un utilisateur est décrit en référence aux figures 7 à 9.

Après que la position et l'orientation du ou des éléments à insérer aient été déterminées, les caractéristiques de ces éléments sont, le cas échéant, estimées (étape 215). Il peut s'agir, par exemple, de déterminer le diamètre et la longueur des éléments à utiliser, ces derniers pouvant être, à titre d'illustration, des vis pédiculaires. Ces caractéristiques peuvent être déterminées automatiquement ou peuvent être déterminées par un utilisateur. Lorsqu'elles sont déterminées par un utilisateur, une vérification est, de préférence, effectuée (étape 220). Une telle vérification peut notamment comprendre l'affichage d'une représentation des éléments sur une représentation de l'organe, à la même échelle, pour permettre à l'utilisateur de vérifier l'adéquation des éléments avec l'organe. Ces représentations peuvent être similaires à celles illustrées sur la figure 1A. A nouveau, une application permettant l'affichage et la manipulation d'objets numériques permet, de préférence, de modifier l'affichage (point de vue, échelle, plan de coupe, etc.).

Comme illustré, il est possible de modifier la position du ou des points d'entrée (retour à la référence 205), la position et l'orientation des éléments à insérer (retour à la référence 210), par exemple pour affiner ces positions et orientations, ou de réestimer les caractéristiques de ces éléments (retour à la référence 215).

Les étapes illustrées sur la figure 2 permettent ainsi, à partir d'un point d'entrée, de déterminer une position et une orientation satisfaisant des contraintes prédéterminées. Le point d'entrée pouvant être choisi de telle sorte à être facilement identifiable lors de l'intervention, l'outil d'aide à une intervention invasive selon l'invention offre des avantages en termes de précision.

La figure 3 illustre un exemple d'étapes pour obtenir un modèle 3D d'un organe humain ou animal ou d'une portion de celui-ci.

Selon l'exemple illustré, une première étape a pour objet l'obtention d'un ensemble de données, par exemple d'images, en coupe, de l'organe humain ou animal, ou d'une partie de celui-ci, dont le modèle 3D est recherché (étape 300). Ces données peuvent être obtenues à l'aide d'un scanner, par exemple un scanner à rayons X, en utilisant une technologie de tomographie numérique, connue sous le nom anglo-saxon de computed tomography (CT).

Ces données sont ensuite combinées pour obtenir un modèle 3D selon des critères prédéterminés, par exemple pour un obtenir un modèle 3D représentatif de la surface externe de l'organe. Il existe de nombreuses solutions pour construire un modèle 3D à partir de données de tomographie numérique (ou CT data en terminologie anglo-saxonne). Les articles connus sous les références suivantes Ney DR, Fishman EK, Magid D, Robertson DD, Kawashima A "Three-dimensional volumetric display of CT data: effect of scan parameters upon image quality", JComput Assist Tomogr, 1991 Sep-Oct;15(5):875-85, PubMed PMID: 1885819, Rothman SL, Geehr RB, Kier EL, Hoffman HB "Multiplanar reconstruction as an aid in CT diagnosis", Neuroradiology, 1978;16:596-7, PubMed PMID: 745768 et Aubry S, Pousse A, Sarliève P, Laborie L, Delabrousse E, Kastler B, "Three-dimensional 3D modeling: First applications in radioanatomy and interventional radiology under CT guidance", J Radiol, 2006 Nov; 87(11 Pt 1):1683-9, French PubMed PMID: 17095963 décrivent certaines de ces méthodes ou certains éléments de celles-ci.

Selon des modes de réalisation, le modèle 3D obtenu peut être amélioré (étape 310), par exemple pour supprimer du bruit. Des exemples d'amélioration d'un modèle 3D sont présentés dans l'article référencé Bibb, R. (2006) "Medical imaging for rapid prototyping", MedicalModelling, 8-31, doi:10.1533/9781845692001.8

Comme décrit précédemment, une région d'intérêt du modèle 3D peut, de façon optionnelle, être sélectionnée (étape 315), de façon automatique selon des critères prédéterminés ou de façon manuelle par un utilisateur. A titre d'illustration, si le modèle 3D correspond à une colonne vertébrale, une région d'intérêt peut correspondre à une ou plusieurs vertèbres, comme illustré sur les figures 5A et 5B.

Selon des modes de réalisation, un contrôle de la région d'intérêt est effectué (étape non représentée sur la figure 3) pour vérifier que celle-ci comprend des éléments discriminants permettant d'identifier un point d'intérêt. A titre d'illustration, il peut ainsi être vérifié qu'une portion d'un modèle 3D de colonne vertébrale comprend au moins la deuxième vertèbre cervicale (C2), la première ou la douzième vertèbre thoracique (T1 ou T12) ou la cinquième vertèbre lombaire (L5).

La figure 4 illustre un exemple d'étapes pour déterminer un axe d'insertion d'un élément dans un organe humain ou animal, par exemple un axe d'un support tel qu'une vis pédiculaire.

Une première étape vise ici à définir une variable contrôlant directement ou indirectement la direction de l'axe d'insertion de l'élément dans l'organe (étape 400). A titre d'illustration, cette direction peut être définie par l'intersection de deux plans, par exemple un premier plan comprenant une droite reliant deux points d'entrée et un second plan comprenant une droite perpendiculaire au premier plan et comprenant l'un de ces deux points d'entrée. Dans ce cas, la variable peut être définie par deux angles associés à deux axes de rotation, l'un de ces axes de rotation étant la droite passant par les deux points d'entrée et l'autre axe de rotation étant la droite perpendiculaire au premier plan et passant par l'un des points d'entrée. Ces angles sont définis par rapport à une référence prédéterminée. Ces deux axes de rotation sont illustrés sur la figure 7.

Dans une étape suivante, une ou plusieurs vues en coupe du modèle 3D peuvent être affichées pour aider un utilisateur à choisir une direction de l'axe d'insertion de l'élément dans l'organe (étape 405). Il peut s'agir, par exemple, de vues en coupe selon les premier et second plans définis précédemment. Un exemple de telles représentations est illustré aux figures 8 et 9. L'utilisateur peut alors faire varier les plans de coupe (étape 410), par exemple en les faisant tourner autour des axes de rotation décrits précédemment, par exemple à l'aide d'une molette d'une souris (l'une des rotations pouvant, par exemple, être effectuée directement avec la molette et l'autre en appuyant simultanément sur une touche, par exemple la touche majuscule). Lorsque l'utilisateur est satisfait de la direction de l'axe d'insertion de l'élément dans l'organe, il peut la valider (étape 415).

Le processus de détermination des axes d'insertion d'éléments dans l'organe peut également être automatique, par exemple en faisant varier la ou les variables contrôlant directement ou indirectement la direction d'un axe d'insertion d'un élément dans l'organe entre des valeurs données et en identifiant une position optimale selon des critères prédéterminés ou une position satisfaisant des critères prédéterminés.

A titre d'illustration, l'angle de rotation du premier plan comprenant la droite passant par deux points d'entrée peut varier de -θ à θ, par exemple de -35° à 35° par rapport à un plan de référence (par exemple un plan horizontal dans le cas de vis pédiculaires). De même, l'angle de rotation du second plan, comprenant une droite perpendiculaire au premier plan, peut varier de -ϕ à ϕ, par exemple de -20° à 20°, par rapport à un plan de référence (par exemple un plan vertical perpendiculaire au premier plan).

Les figures 5A et 5B illustrent un exemple de modèle 3D d'une colonne vertébrale, permettant à un utilisateur de déterminer un ou plusieurs points d'entrée puis de déterminer un ou plusieurs axes d'insertion de vis pédiculaires.

Comme illustré, le modèle est ici associé à un repère, par exemple un repère cartésien dans lequel l'axe x est un axe horizontal s'étendant vers l'avant, l'axe y est un axe horizontal perpendiculaire à l'axe x et l'axe z est un axe vertical s'étendant vers le haut. Naturellement, un autre repère peut être utilisé, par exemple un repère lié à une vertèbre. Selon un mode de réalisation, le modèle 3D est représenté sur un écran, comme illustré sur la figure 5A. De préférence, des fonctions permettent de déplacer le modèle 3D sur l'écran, de modifier son orientation et/ou de modifier son échelle.

Selon un mode de réalisation particulier, un utilisateur peut sélectionner une région d'intérêt, par exemple à l'aide d'un parallélépipède 505, d'un cylindre ou de tout autre forme tridimensionnelle dont, en particulier, la forme, la taille, la position et/ou l'orientation peuvent être ajustées par un utilisateur ou de façon automatique. La portion du modèle 3D 510 ainsi définie peut être affichée, comme illustré sur la figure 5B. A nouveau, l'utilisateur peut, de préférence, déplacer cette portion sur l'écran, modifier son orientation et/ou modifier son échelle. Il peut également, de préférence, exécuter un algorithme de traitement d'image pour améliorer la qualité du rendu de la représentation, par exemple pour réduire le bruit.

La représentation du modèle 3D ou d'une portion de celui-ci permet de déterminer un ou plusieurs points d'entrée, par exemple les points d'entrée 515-1 et 515-2. A partir de ces points d'entrée, il est possible de déterminer des axes d'insertion de vis pédiculaires comme décrit plus en détail en référence aux figures 7, 8 et 9.

Pour faciliter la détermination de points d'entrée et/ou d'axes d'insertion de vis pédiculaires, plusieurs vues peuvent être présentées simultanément, comme illustré sur les figures 6A et 6B.

Les figures 6A et 6B représentent un exemple d'une interface graphique pouvant être utilisée par un utilisateur pour déterminer des points d'entrée et des axes d'insertion de vis pédiculaires.

Plus précisément, la figure 6A illustre un exemple d'interface graphique 600 pour déterminer un point d'entrée. Comme illustré, l'interface graphique 600 comprend ici plusieurs zones parmi lesquelles une zone 605 comprenant des icônes permettant d'accéder à des menus, une zone de texte 610 pour afficher ou saisir, par exemple, des informations ou des instructions et quatre zones graphiques 615 à 630. Selon cet exemple, la zone 615 comprend une représentation du modèle 3D ou d'une portion de celui-ci tandis que les zones 620, 625 et 630 comprennent respectivement des vues en coupe du modèle 3D, par exemple selon des plans xy, xz et yz comprenant un point donné pouvant être déplacé par l'utilisateur, par exemple un point pouvant être sélectionné par l'utilisateur comme point d'entrée. Pour déplacer ce point, l'utilisateur peut, par exemple, sélectionner l'une des zones 620, 625 ou 630 et déplacer le point à l'aide d'une souris, d'une molette de souris ou de touches de clavier (par exemple les touches gauche, droite, haut et bas). Lorsque la position du point est modifiée, les représentations sur l'écran le sont également, avantageusement immédiatement (i.e. en temps réel). Comme décrit précédemment, ce point (ici présenté sous forme d'une étoile pour être visible) peut être sélectionné par l'utilisateur comme point d'entrée.

La figure 6B illustre un exemple d'interface graphique 600' pour déterminer un axe d'insertion d'un élément dans un organe, par exemple d'une vis pédiculaire dans une vertèbre. Outre des zones comprenant des icônes ou des emplacements pour afficher ou saisir des informations ou des instructions, similaires à celles représentées sur la figure 6A, l'interface graphique comprend ici deux zones graphiques 635 et 640.

Selon un mode de réalisation particulier, chacune des zones 635 et 640 comprend une vue en coupe du modèle 3D, ces vues étant obtenues selon deux plans orthogonaux dont l'intersection représente l'axe d'insertion d'une vis pédiculaire. Ainsi, par exemple, la zone 635 comprend une vue en coupe selon un premier plan comprenant deux points d'entrée, par exemple des points d'entrée situés sur deux lames opposées d'une même vertèbre, tandis que la zone 640 comprend une vue en coupe selon un second plan, comprenant une droite perpendiculaire au premier plan et comprenant le point d'entrée associé à l'axe d'insertion en cours de détermination. Selon un mode de réalisation, l'utilisateur peut sélectionner la zone 635 ou la zone 640 et utiliser une molette ou deux flèches pour augmenter ou diminuer l'angle du plan correspondant à la coupe visualisée.

L'axe d'insertion 645 et/ou l'élément à insérer sont représentés sur les vues en coupe pour aider l'utilisateur à déterminer cet axe.

La figure 7 illustre schématiquement un exemple d'une solution pour déterminer un axe d'insertion d'un élément dans un organe, par exemple d'une vis pédiculaire dans une vertèbre.

Selon cet exemple, un axe d'insertion est défini par une droite formée par l'intersection de deux plans et comprenant un point d'entrée précédemment défini de l'élément à insérer. Comme illustré sur la figure 7, l'axe d'insertion en cours de détermination est ici l'axe 700, résultant de l'intersection des plans 705 et 710, comprenant le point d'entrée 715. Pour des raisons de clarté, seul le plan 705 comprend une représentation d'une vue en coupe de l'organe, ici une vertèbre.

A titre d'illustration, le plan 705 est un plan comprenant la droite 725 passant par le point d'entrée 715 et un autre point d'entrée, ici le point d'entrée 720. Le plan 705 peut ainsi tourner autour de l'axe 725 et former un angle θ par rapport à un plan de référence, par exemple le plan perpendiculaire à la colonne vertébral et comprenant les points d'entrée 715 et 720.

Toujours à titre d'illustration, le plan 710 est un plan comprenant une droite 730 perpendiculaire au plan 705 et comprenant le point d'entrée 715. Le plan 720 peut ainsi tourner autour de l'axe 730 (perpendiculaire au plan 705 et comprenant le point d'entrée 715). Il peut former un angle ϕ par rapport à un plan de référence, par exemple le plan vertical perpendiculaire à l'axe 725.

Pour déterminer l'axe d'insertion d'un élément, un utilisateur (ou un algorithme) peut modifier les valeurs des angles θ et ϕ afin de visualiser (ou analyser) les vues en coupe selon les deux plans définissant l'axe d'insertion. Comme décrit précédemment, la valeur des angles θ et ϕ peut notamment être modifiée à l'aide de molettes, de touches ou d'une combinaison d'une molette et d'une touche.

L'axe d'insertion peut ainsi être défini, par exemple, par les angles θ et ϕ et des plans de référence.

La figure 8 illustre un exemple de vues en coupe obtenues lors de la rotation d'un plan passant par deux points d'entrée, selon le mécanisme illustré sur la figure 7.

Les vues situées sur la partie gauche sont des vues en coupe selon le plan 705 passant par les points d'entrée 715 et 720 tandis que les vues situées sur la partie droite sont des vues en coupe selon le plan 710, comprenant la droite 730 (perpendiculaire au plan 705 et passant par le point d'entrée 715). Les ensembles de vues référencés (a), (b) et (c) correspondent à différentes valeurs de l'angle θ.

La figure 9 illustre un exemple de vues en coupe obtenues lors de la rotation d'un plan perpendiculaire à un plan passant par deux points d'entrée, selon le mécanisme illustré sur la figure 7.

A nouveau, les vues situées sur la partie gauche sont des vues en coupe selon le plan 705 passant par les points d'entée 715 et 720 tandis que les vues situées sur la partie droite sont des vues en coupe selon le plan 710, comprenant la droite 730 (perpendiculaire au plan 705 et passant par le point d'entrée 715). Les ensembles de vues référencés (a), (b) et (c) correspondent à différentes valeurs de l'angle ϕ.

Ainsi, en faisant successivement varier les angles θ et ϕ, il est possible de trouver un axe d'insertion permettant l'insertion d'un élément dans un organe à partir d'un point d'entrée, respectant des critères prédéterminés. Pour aider l'utilisateur, une représentation de l'élément à insérer peut être ajoutée aux vues en coupe, la position de cette représentation étant déterminée par le point d'entrée et l'axe d'insertion considérés.

Comme décrit précédemment, les valeurs des angles θ et ϕ peuvent être modifiées par un utilisateur pour lui permettre, à partir des vues en coupe, de déterminer l'axe d'insertion qui lui semble optimal. Alternativement, les valeurs des angles θ et ϕ peuvent être modifiées de façon automatique, selon des incréments prédéterminés. Pour chaque incrément, une analyse des vues en coupe est effectuée pour déterminer si l'axe d'insertion défini par les angles θ et ϕ satisfait des critères prédéterminés. Si ces critères sont satisfaits, l'algorithme prend fin ou mémorise les valeurs identifiées pour, ultérieurement, comparer les différents axes d'insertion satisfaisant les critères prédéterminés et sélectionner l'axe d'insertion présentant des critères optimaux.

Selon un mode de réalisation particulier, une assistance est fournie à l'utilisateur pour lui permettre de modifier les angles θ et ϕ tout en lui indiquant si les valeurs courantes de ces angles satisfont des critères prédéterminés. Ainsi, par exemple, l'axe d'insertion peut être coloré en vert si l'axe d'insertion répond aux critères prédéterminés et en rouge si ces derniers ne le sont pas. De même, ces couleurs peuvent être plus ou moins intenses selon, par exemple, une quantification de risque associée à une position de l'axe d'insertion.

La figure 10 représente un exemple de dispositif de traitement de l'information permettant de mettre en oeuvre le procédé selon des modes de réalisation de l'invention, notamment d'exécuter toutes ou certaines des étapes décrites en référence aux figures 2, 3 et 4.

Selon l'exemple illustré, le dispositif 1000 comprend une mémoire 1005 pour stocker des instructions permettant la mise en oeuvre du procédé, les données de mesures reçues et des données temporaires pour réaliser différentes étapes du procédé tel que décrit précédemment.

Le dispositif comporte en outre un circuit 1010. Ce circuit peut être, par exemple :
- un processeur apte à interpréter des instructions sous la forme de programme informatique, ou
- une carte électronique dont des étapes du procédé de l'invention sont implémentées dans des éléments matériels ou encore
- une puce électronique programmable comme une puce FPGA (pour « Field-Programmable Gate Array » en terminologie anglo-saxonne), un SOC (pour « System On Chip » en terminologie anglo-saxonne) ou un ASIC (pour « Application Specific Integrated Circuit » en terminologie anglo-saxonne).

Les SOCs et systèmes sur puce sont des systèmes embarqués qui intègrent tous les composants d'un système électronique dans une puce unique.

Un ASIC est un circuit électronique spécialisé qui regroupe des fonctionnalités sur mesure pour une application donnée. Les ASIC sont généralement configurés lors de leur fabrication.

Les circuits logiques programmables de type FPGA sont des circuits électroniques reconfigurables par un utilisateur.

Le dispositif 1000 comporte ici une interface d'entrée 1015 pour la réception de données de mesures, par exemple de données CT permettant la construction d'un modèle 3D, et une interface de sortie 1020. Enfin, il peut comporter, pour permettre une interaction aisée avec un utilisateur, un écran 1025, un clavier 1030 et une souris 1035, de préférence pourvue d'une roulette. Bien entendu, le clavier est facultatif, notamment dans le cadre d'un ordinateur ayant la forme d'une tablette tactile, par exemple.

En fonction du mode de réalisation, le dispositif 1000 peut être un ordinateur, un réseau d'ordinateurs, un composant électronique ou un autre appareil comportant un processeur couplé de manière opérationnelle à une mémoire, ainsi que, selon le mode de réalisation choisi, une unité de stockage de données et d'autres éléments matériels associés comme une interface de réseau et un lecteur de support pour lire un support de stockage amovible et écrire sur un tel support (non représentés sur la figure). Le support de stockage amovible peut être, par exemple, un disque compact (CD), un disque vidéo/polyvalent numérique (DVD), un disque flash, une clé USB, etc.

En fonction du mode de réalisation, la mémoire, l'unité de stockage de données ou le support de stockage amovible contient des instructions qui, lorsqu'elles sont exécutées par le circuit 1010, amènent ce circuit 1010 à effectuer ou contrôler les parties interface d'entrée 1015, interface de sortie 1020, stockage de données dans la mémoire 1005 et/ou traitement de données.

Les schémas fonctionnels présentés sur les figures 2, 3 et 4 sont des exemples de programmes dont toutes ou certaines instructions peuvent être réalisées par le dispositif décrit.

Bien entendu, la présente invention ne se limite pas aux formes de réalisation décrites ci-avant à titre d'exemples, elle s'étend à d'autres variantes. D'autres réalisations sont possibles, l'invention étant uniquement définie par les revendications.

En fonction du mode de réalisation choisi, certains actes, actions, évènements ou fonctions de chacune des méthodes décrites dans le présent document peuvent être effectués ou se produire selon un ordre différent de celui dans lequel ils ont été décrits, ou peuvent être ajoutés, fusionnés ou bien ne pas être effectués ou ne pas se produire, selon le cas. En outre, dans certains modes de réalisation, certains actes, actions ou évènements sont effectués ou se produisent concurremment et non pas successivement.

## Revendications

1. Procédé d'aide à une intervention invasive sur un organe humain ou animal, ladite intervention comprenant l'insertion d'au moins un élément dans ledit organe, le procédé comprenant :
a. l'obtention d'un modèle tridimensionnel d'au moins une partie dudit organe ;
b. la détermination sur le modèle tridimensionnel obtenu d'au moins un point d'entrée dudit au moins un élément ;
c. la détermination d'un axe d'insertion dudit au moins un élément dans ledit organe, en fonction dudit modèle tridimensionnel, dudit au moins un point d'entrée déterminé et d'une pluralité d'axes distincts passant par ledit au moins un point d'entrée, ladite pluralité d'axes étant définie par l'intersection d'un premier et d'un second plans, ledit premier et ledit second plans comprenant ledit au moins un point d'entrée,
d. l'affichage d'une vue en coupe dudit modèle tridimensionnel selon ledit premier plan, ledit premier plan formant un premier angle par rapport à une première référence liée audit modèle tridimensionnel,
**caractérisé en ce que** le procédé comprend:
e. la modification de la valeur dudit premier angle, l'affichage d'une vue en coupe selon ledit premier plan étant adapté à ladite modification dudit premier angle.

2. Procédé selon la revendication 1 comprenant en outre la construction dudit modèle tridimensionnel.

3. Procédé selon la revendication 1 ou la revendication 2 comprenant l'affichage d'une représentation d'au moins une partie dudit modèle tridimensionnel, l'affichage d'au moins une partie dudit modèle tridimensionnel permettant l'affichage et/ou la détermination d'au moins un point d'entrée.

4. Procédé selon l'une quelconque des revendications 1 à 3 selon lequel au moins deux points d'entrées sont déterminés, ledit premier plan comprenant une droite passant par lesdits au moins deux points d'entrée.

5. Procédé selon la revendication 4 selon lequel ledit second plan comprend une droite perpendiculaire audit premier plan.

6. Procédé selon l'une quelconque des revendications 1 à 5 comprenant en outre l'affichage d'une vue en coupe dudit modèle tridimensionnel selon ledit second plan, ledit second plan formant un second angle par rapport à une seconde référence liée audit modèle tridimensionnel.

7. Procédé selon la revendication 6 selon lequel la valeur dudit second angle est modifiée, l'affichage d'une vue en coupe selon ledit second plan étant adapté à ladite modification dudit second angle.

8. Procédé selon l'une quelconque des revendications 1 à 7 comprenant le déplacement d'au moins un point d'entrée défini sur ledit modèle tridimensionnel.

9. Procédé selon l'une quelconque des revendications 1 à 8 comprenant une mesure d'un diamètre et/ou d'une longueur d'un élément à insérer selon un point d'entrée déterminé, un axe d'insertion déterminé et ledit modèle tridimensionnel.

10. Procédé selon l'une quelconque des revendications 1 à 9 selon lequel ledit organe est une vertèbre et ledit élément à insérer est une vis pédiculaire.

11. Produit programme informatique comportant des instructions pour la mise en oeuvre de chacune des étapes du procédé selon l'une des revendications 1 à 10, lorsque ce programme est exécuté par un processeur.

12. Support d'enregistrement non transitoire lisible par un ordinateur sur lequel est enregistré un programme pour la mise en oeuvre de chacune des étapes du procédé selon l'une des revendications 1 à 10 lorsque ce programme est exécuté par un processeur.

13. Dispositif comprenant une unité de calcul configurée pour exécuter chacune des étapes du procédé selon l'une des revendications 1 à 10.

## Patentansprüche

1. Verfahren zur Unterstützung eines invasiven Eingriffs an einem menschlichen oder tierischen Organ, wobei der Eingriff das Einführen mindestens eines Elements in das Organ umfasst, wobei das Verfahren Folgendes umfasst:
a. Erhalten eines dreidimensionalen Modells von mindestens einem Teil des Organs;
b. Bestimmen, anhand des erhaltenen dreidimensionalen Modells, mindestens eines Eintrittspunkts des mindestens einen Elements;
c. Bestimmen einer Einführachse für das mindestens eine Element in das Organ gemäß dem dreidimensionalen Modell, des mindestens einen bestimmten Eintrittspunkts und einer Vielzahl verschiedener Achsen, die durch den mindestens einen Eintrittspunkt verlaufen, wobei die Vielzahl von Achsen durch den Schnittpunkt einer ersten und einer zweiten Ebene definiert ist, wobei die erste und die zweite Ebene den mindestens einen Eintrittspunkt umfassen,
d. Anzeigen einer Querschnittsansicht des dreidimensionalen Modells in der ersten Ebene, wobei die erste Ebene einen ersten Winkel im Verhältnis zu einem ersten Bezugspunkt bildet, der mit dem dreidimensionalen Modell verknüpft ist,
**dadurch gekennzeichnet, dass** das Verfahren Folgendes umfasst:
e. Modifizieren des Werts des ersten Winkels, wobei die Anzeige einer Querschnittsansicht in der ersten Ebene an das Modifizieren des ersten Winkels angepasst wird.

2. Verfahren nach Anspruch 1, das ferner das Erstellen des dreidimensionalen Modells umfasst.

3. Verfahren nach Anspruch 1 oder Anspruch 2, das das Anzeigen einer Darstellung mindestens eines Teils des dreidimensionalen Modells umfasst, wobei das Anzeigen mindestens eines Teils des dreidimensionalen Modells das Anzeigen und/oder das Bestimmen mindestens eines Eintrittspunkts ermöglicht.

4. Verfahren nach einem der Ansprüche 1 bis 3, wobei mindestens zwei Eintrittspunkte bestimmt werden, wobei die erste Ebene eine gerade Linie umfasst, die durch die mindestens zwei Eintrittspunkte verläuft.

5. Verfahren nach Anspruch 4, wobei die zweite Ebene eine gerade Linie senkrecht zur ersten Ebene umfasst.

6. Verfahren nach einem der Ansprüche 1 bis 5, das ferner das Anzeigen einer Querschnittsansicht des dreidimensionalen Modells in der zweiten Ebene umfasst, wobei die zweite Ebene einen zweiten Winkel im Verhältnis zu einem zweiten Bezugspunkt bildet, der mit dem dreidimensionalen Modell verknüpft ist.

7. Verfahren nach Anspruch 6, wobei der Wert des zweiten Winkels geändert wird, wobei das Anzeigen einer Querschnittsansicht in der zweiten Ebene an das Modifizieren des zweiten Winkels angepasst wird.

8. Verfahren nach einem der Ansprüche 1 bis 7, das das Verschieben mindestens eines auf dem dreidimensionalen Modell definierten Eintrittspunkts umfasst.

9. Verfahren nach einem der Ansprüche 1 bis 8, das ein Messen eines Durchmessers und/oder einer Länge eines einzusetzenden Elements gemäß einem bestimmten Eintrittspunkt, einer bestimmten Einführungsachse und dem dreidimensionalen Modell umfasst.

10. Verfahren nach einem der Ansprüche 1 bis 9, wobei das Organ ein Wirbel ist und das einzuführende Element eine Pedikelschraube ist.

11. Computerprogrammprodukt, das Anweisungen zum Durchführen jeder der Schritte des Verfahrens nach einem der Ansprüche 1 bis 10 umfasst, wenn dieses Programm von einem Prozessor ausgeführt wird.

12. Computerlesbares, nichtflüchtiges Aufzeichnungsmedium, auf dem ein Programm zum Durchführen jedes der Schritte des Verfahrens nach einem der Ansprüche 1 bis 10 aufgezeichnet ist, wenn dieses Programm von einem Prozessor ausgeführt wird.

13. Vorrichtung, die eine Recheneinheit umfasst, die dazu konfiguriert ist, jeden der Schritte des Verfahrens nach einem der Ansprüche 1 bis 10 auszuführen.

## Claims

1. A method for assisting an invasive procedure on a human or animal organ, said procedure comprising the insertion of at least one element into said organ, the method comprising:
a. obtaining a three-dimensional model of at least one portion of said organ;
b. determining at least one entry point of said at least one element on the obtained three-dimensional model;
c. determining an axis of insertion of said at least one element in said organ, depending on said three-dimensional model, of said at least one determined entry point and of a plurality of distinct axes passing through said at least one entry point, said plurality of axes being defined by the intersection of a first plane and a second plane, said first and second planes comprising said at least one entry point,
d. displaying a sectional view of said three-dimensional model according to said first plane, said first plane forming a first angle relative to a first reference related to said three-dimensional model,
**characterised in that** the method comprises:
e. changing the value of said first angle, displaying a sectional view along said first plane being adapted to said change in said first angle.

2. The method according to claim 1, further comprising constructing said three-dimensional model.

3. The method according to claim 1 or claim 2, comprising displaying a representation of at least one portion of said three-dimensional model, displaying at least one portion of said three-dimensional model allowing displaying and/or determining at least one entry point.

4. The method according to any one of claims 1 to 3, according to which at least two entry points are determined, said first plane comprising a straight line passing through said at least two entry points.

5. The method according to claim 4, according to which said second plane comprises a straight line which is perpendicular to said first plane.

6. The method according to any one of claims 1 to 5, further comprising displaying a sectional view of said three-dimensional model according to said second plane, said second plane forming a second angle relative to a second reference related to said three-dimensional model.

7. The method according to claim 6, according to which the value of said second angle is changed, the display of a sectional view according to said second plane being adapted to said change in said second angle.

8. The method according to any one of claims 1 to 7, comprising displacing at least one entry point defined on said three-dimensional model.

9. The method according to any one of claims 1 to 8, comprising measuring a diameter and/or a length of an element to be inserted according to a determined entry point, a determined insertion axis and said three-dimensional model.

10. The method according to any one of claims 1 to 9, according to which said organ is a vertebra and said element to be inserted is a pedicle screw.

11. A computer program product including instructions for implementing each of the steps of the method according to one of claims 1 to 10, when this program is executed by a processor.

12. A non-transitory computer-readable recording medium on which a program for implementing each of the steps of the method is recorded according to one of claims 1 to 10 when this program is executed by a processor.

13. A device comprising a calculation unit configured to execute each of the steps of the method according to one of claims 1 to 10.
